# FASCICULE DE BREVET EUROPEEN

(11) **EP 4 051 340 B1**
(45) Date de publication et mention de la délivrance du brevet: **10.12.2025**
(21) Numéro de dépôt: 20797132.6
(22) Date de dépôt: 30.10.2020
(51) Int. Cl.: A61M 5/168, A61M 5/315, A61M 15/00, A61M 15/08, B05B 11/00, G16H 20/13, G16H 40/63

(54) **DISPOSITIF D'ASSISTANCE À L'UTILISATION D'UN DISPOSITIF DE DISTRIBUTION D'UN PRODUIT**
VORRICHTUNG ZUR UNTERSTÜTZUNG DER VERWENDUNG EINER VORRICHTUNG ZUR ABGABE EINES PRODUKTS
DEVICE FOR ASSISTING IN THE USE OF A DEVICE FOR DISPENSING A PRODUCT

(30) Priorité: 31.10.2019 FR 1912305
(43) Date de publication de la demande: 07.09.2022
(73) Titulaire: NEMERA LA VERPILLIERE, 38290 La Verpillière (FR)
(72) Inventeur: DECOCK, Thierry, 69007 Lyon (FR); GUIMARD, Lenaic, 38500 Saint-Cassien (FR); DELAY, Adrien, 34270 Vacquières (FR); POULAIN, Eric, 01120 Montluel (FR); VERNANT, Olivier, 38280 Anthon (FR)
(74) Mandataire: LLR
(86) Numéro de dépôt international: PCT/EP2020/080567
(87) Numéro de publication internationale: WO 2021/084097

(56) Documents cités:
- WO-A1-2016/100564
- WO-A1-2016/128207
- US-A- 5 284 133

## Description

L'invention concerne un dispositif d'assistance à l'utilisation d'un dispositif de distribution de produit, ainsi qu'un procédé d'utilisation d'un dispositif de distribution de produit.

On connaît déjà dans l'état la technique, notamment d'après le document WO 2016/100564, un dispositif d'assistance à l'utilisation d'un dispositif de distribution de produit comprenant des moyens de verrouillage qui empêchent, après la distribution d'une dose complète de produit, la délivrance d'une seconde dose avant qu'un intervalle de temps prévu en fonction de la posologie du médicament ne se soit écoulé. On évite ainsi un surdosage ou l'utilisation du dispositif par une personne autre que le patient entre les temps prévus pour l'administration du produit. Cela est particulièrement avantageux pour des médicaments tels que les antidouleurs, notamment les antidouleurs à base d'opiacés.

Un tel dispositif présente des inconvénients. Notamment, le dispositif d'assistance est verrouillé après la délivrance d'une dose complète de produit pendant toute la période normalement prévue entre les prises de doses, ce qui ne laisse pas la possibilité au patient de prendre une double dose de produit, par exemple dans le cas où la douleur serait trop importante et pas assez soulagée avec la première dose. En outre, une fois déverrouillé, une distribution de produit doit avoir lieu pour que le reverrouillage du dispositif soit possible.

Le document US 5 284 133 A décrit un dispositif d'assistance à l'utilisation d'un dispositif de distribution d'un produit selon le préambule de la revendication 1.

L'invention a notamment pour but de fournir un dispositif d'assistance à l'utilisation d'un dispositif de distribution de produit permettant de surmonter au moins une partie de ces inconvénients.

À cet effet, l'invention a notamment pour objet un dispositif d'assistance à l'utilisation d'un dispositif de distribution d'un produit selon la revendication 1.

Un mode de réalisation a notamment pour objet un dispositif d'assistance à l'utilisation d'un dispositif de distribution d'un produit, le dispositif d'assistance comprenant : - des moyens de solidarisation au dispositif de distribution, - des moyens de verrouillage du dispositif de distribution aptes à empêcher ou permettre une distribution de produit, et - des moyens de mesure d'une quantité de produit distribuée par le dispositif de distribution, le dispositif d'assistance étant agencé pour que les moyens de verrouillage restent déverrouillés pendant une période de temps prédéterminée à partir d'un actionnement prédéterminé du dispositif de distribution puis se verrouillent automatiquement à l'issue de la période de temps prédéterminée, et en ce qu'il est agencé en outre pour que les moyens de verrouillage se verrouillent automatiquement lorsqu'une quantité de produit distribuée pendant la période de temps prédéterminée atteint ou dépasse un seuil prédéterminé.

Ainsi, une fois les moyens de verrouillage déverrouillés, si aucune distribution de produit n'a lieu pendant la période de temps prédéterminée, par exemple parce que l'utilisateur ne souhaite finalement pas obtenir du produit ou parce que le déverrouillage a eu lieu accidentellement, les moyens de verrouillage se verrouillent automatiquement ce qui évite que du produit soit gaspillé. En outre, cela réduit le risque, dans le cas où les moyens de verrouillage seraient déverrouillés accidentellement, qu'une quantité de produit non souhaitée soit distribuée.

Un actionnement prédéterminé du dispositif d'assistance peut notamment correspondre à un déverrouillage des moyens de verrouillage. Ce déverrouillage peut par exemple être réalisé par authentification d'une empreinte digitale à l'aide d'un capteur d'empreinte digitale ou par l'entrée d'un code prédéterminé.

Dans un autre mode de réalisation possible, l'actionnement du dispositif d'assistance via un déverrouillage des moyens de verrouillage peut être réalisé via une authentification de la voix et/ou d'une commande vocale prédéterminée et émise par l'utilisateur.

Les moyens de verrouillage sont soit verrouillés de manière à empêcher une distribution de produit, soit déverrouillés de manière à permettre une distribution de produit. En position verrouillée, les moyens de verrouillage verrouillent le dispositif de distribution qui ne peut pas être actionné. En position déverrouillée, les moyens de verrouillage déverrouillent le dispositif de distribution qui peut donc être actionné.

Un actionnement prédéterminé du dispositif de distribution peut notamment correspondre à un actionnement complet du dispositif de distribution, c'est-à-dire un actionnement entrainant la distribution d'une dose maximale de produit pouvant être délivrée en une seule fois par le dispositif de distribution. Il peut par exemple s'agir d'un actionnement complet d'une pompe du dispositif de distribution. On peut également prévoir que l'actionnement prédéterminé ne correspond pas à un actionnement complet mais à un actionnement partiel, correspondant par exemple à une course prédéterminée d'un applicateur du dispositif de distribution.

On peut prévoir que le seuil de quantité de produit pouvant être distribué pendant la période de temps prédéterminée correspond à une dose de produit devant être administré lors d'une prise du produit. Par exemple, dans le cas d'un médicament, il s'agit de la dose que doit prendre le patient lors de chaque prise de médicament. On peut prévoir que cette dose est atteinte par un ou plusieurs actionnements du dispositif de distribution. Entre les actionnements nécessaires à la délivrance d'une dose, les moyens de verrouillage ne se verrouillent pas automatiquement.

Le fait que les moyens de verrouillage se verrouillent automatiquement si, pendant la période de temps prédéterminée, une quantité de produit distribuée atteint ou dépasse le seuil prédéterminé réduit le risque de surdosage, notamment dans le cas où le produit est un médicament, en empêchant qu'une dose supérieure à une dose maximale de produit autorisée puisse être délivrée. En outre, on réduit le risque de gaspillage de produit dans le cas où le dispositif de distribution serait déverrouillé par accident, par exemple dans un sac ou dans une poche, et que le dispositif de distribution serait actionné à plusieurs reprises accidentellement pendant la période de temps prédéterminée. L'actionnement du dispositif de distribution pourrait ainsi avoir lieu seulement jusqu'à ce que le seuil prédéterminé de produit soit atteint plutôt que jusqu'à l'issue de la période de temps prédéterminée.

De manière avantageuse, la période de temps prédéterminée est comprise entre 1 et 20 minutes, de préférence entre 5 et 10 minutes.

Cette période de temps prédéterminée est ainsi suffisamment longue pour laisser le temps à l'utilisateur, après avoir déverrouillé les moyens de verrouillage, d'actionner le dispositif de distribution, et suffisamment courte pour conserver les avantages précédemment cités liés au reverrouillage à l'issue de la période de temps prédéterminée (réduction du risque de surdosage et de distribution accidentelle de produit).

Avantageusement, la période de temps prédéterminée étant une première période de temps prédéterminée, le dispositif d'assistance comprend en outre des moyens de contrôle aptes à empêcher ou permettre un déverrouillage des moyens de verrouillage, les moyens de contrôle étant configurés pour permettre un déverrouillage des moyens de verrouillage pendant une deuxième période de temps prédéterminée à partir du verrouillage automatique des moyens de verrouillage,
le dispositif d'assistance étant agencé pour que les moyens de verrouillage se verrouillent automatiquement à l'issue de la deuxième période de temps et pour que les moyens de verrouillage se verrouillent automatiquement lorsqu'une quantité de produit distribuée pendant la deuxième période de temps atteint ou dépasse le seuil prédéterminé.

Ainsi, le dispositif laisse à l'utilisateur le temps de vérifier si le produit a fait suffisamment effet et lui donne l'opportunité de déverrouiller à nouveau les moyens de verrouillage afin d'obtenir une dose de produit. Cela est particulièrement avantageux dans le cas où le produit est un médicament de type antidouleurs, par exemple à base d'opiacés, puisque l'utilisateur peut mesurer directement l'efficacité du médicament par le soulagement plus ou moins important de la douleur ressentie. De cette façon, si la douleur est plus importante que d'habitude ou si la première dose de médicament n'a pas ou peu soulagé la douleur, le dispositif d'assistance permet à l'utilisateur de prendre au moins une deuxième dose pendant la deuxième période de temps. À nouveau, le risque qu'un tiers auquel le produit n'est pas destiné puisse utiliser le dispositif de distribution après que l'utilisateur autorisé l'ait déverrouillé est réduit puisque les moyens de verrouillage se verrouillent automatiquement à l'issue de la deuxième période de temps. De plus, le fait que les moyens de verrouillage se verrouillent automatiquement lorsque le seuil prédéterminé de quantité de produit est atteint ou dépassé pendant la deuxième période de temps évite que l'utilisateur puisse commander à volonté la distribution de doses de produit après le déverrouillage des moyens de verrouillage, ce qui réduit le risque de surdosage.

Avantageusement, la deuxième période de temps est comprise entre 1 et 60 minutes, de préférence entre 10 et 30 minutes.

Il s'agit du temps que met en moyenne un produit tel qu'un antidouleur pour faire effet. Ce temps est donc suffisamment long pour permettre à l'utilisateur de se rendre compte si le produit a fait effet et suffisamment court pour conserver les avantages précédemment cités.

De manière avantageuse, les moyens de contrôle sont configurés pour empêcher un déverrouillage des moyens de verrouillage pendant une troisième période de temps prédéterminée à partir du verrouillage automatique des moyens de verrouillage à l'issue de la deuxième période de temps ou lorsque la quantité de produit distribuée pendant la deuxième période de temps a atteint ou dépassé le seuil prédéterminé.

Ainsi, suite à la délivrance d'une deuxième dose de produit, les moyens de contrôle empêchent un déverrouillage des moyens de verrouillage, et donc une distribution de produit, pendant la troisième période de temps. Cette troisième période de temps correspond par exemple au temps minimum qui doit s'écouler entre une prise de dose (éventuellement une double dose) et la suivante. On réduit ainsi encore le risque de surdosage. On peut prévoir que cette troisième période de temps est comprise entre une et huit heures, par exemple entre deux et six heures, voire entre deux et quatre heures.

Avantageusement, les moyens de mesure de la quantité de produit distribuée comprennent un compteur d'un nombre d'actionnement prédéterminé du dispositif de distribution.

Il s'agit d'une façon simple de mesurer la quantité de produit distribué. En effet, dans le cas, par exemple, où le dispositif de distribution fonctionne à l'aide d'une pompe, un actionnement du dispositif de distribution entraine la distribution d'une quantité connue de produit. Ainsi, la quantité de produit distribuée peut être facilement déduite du nombre d'actionnements du dispositif de distribution.

Dans un mode de réalisation, la période de temps prédéterminée étant une première période de temps, le dispositif d'assistance comprend en outre des moyens de contrôle aptes à empêcher ou permettre un déverrouillage des moyens de verrouillage, les moyens de contrôle étant configurés pour permettre un déverrouillage des moyens de verrouillage pendant une deuxième période de temps prédéterminée à partir du verrouillage automatique des moyens de verrouillage,
le dispositif d'assistance étant agencé pour que les moyens de verrouillage se verrouillent automatiquement à l'issue de la deuxième période de temps et pour que les moyens de verrouillage se verrouillent automatiquement lorsqu'une quantité de produit distribuée pendant la deuxième période de temps atteint ou dépasse le seuil prédéterminé.

On peut prévoir que la deuxième période de temps est comprise entre 1 et 60 minutes, de préférence entre 10 et 30 minutes.

On peut prévoir que les moyens de contrôle sont configurés pour empêcher un déverrouillage des moyens de verrouillage pendant une troisième période de temps prédéterminée à partir du verrouillage automatique des moyens de verrouillage à l'issue de la deuxième période de temps ou lorsque la quantité de produit distribuée pendant la deuxième période de temps a atteint ou dépassé le seuil prédéterminé.

Dans une autre configuration de l'invention, les moyens de mesure peuvent intégrer un capteur de niveau de remplissage du dispositif de distribution. En effet, dans le cas, par exemple, où le dispositif de distribution comprend un réservoir content le produit à distribuer et si le niveau initial de remplissage de produit est connu, il est possible via le capteur de niveau de remplissage de connaître la quantité de produit à l'instant de la mesure. Ainsi la quantité de produit distribuée peut être facilement déduite.

On peut prévoir que les moyens de mesure de la quantité de produit distribuée comprennent, de manière alternative ou additionnelle, d'autres éléments permettant de mesurer la quantité de produit distribuée et qui sont connus en soi.

De même, dans un autre mode de réalisation de l'invention, il est possible d'associer aux moyens de mesure, des moyens de collecte et de stockage des informations et données liées à l'utilisateur et son utilisation du dispositif de distribution et/ou du dispositif d'assistance. Ces données peuvent, par exemple, comprendre le nombre d'actionnements du dispositif de distribution et/ou le nombre d'actionnements du dispositif. Enfin, il est possible d'associer ces moyens de collecte et de stockage avec des moyens de communication afin de transférer les informations contenues par les moyens de collecte et de stockage. Ces moyens de communications peuvent ainsi, par exemple, permettre au personnel de santé et/ou à l'utilisateur de récupérer des informations sur la prise du produit, dans le cas où ce dernier correspond à un traitement médical.

De manière avantageuse, le dispositif d'assistance comprend des moyens de signalement configurés pour émettre au moins un signal donnant une information sur l'écoulement d'au moins une des première, deuxième et troisième périodes de temps.

Ainsi, l'utilisateur est mieux informé du temps qu'il lui reste pour prendre une première dose ou une dose supplémentaire de produit avant que les moyens de verrouillage ne se verrouillent ou qu'un déverrouillage du dispositif ne soit plus possible et n'est donc pas pris au dépourvu. Grâce à l'information sur la troisième période de temps, l'utilisateur est également mieux informé du temps à attendre avant de pouvoir à nouveau déverrouiller le dispositif pour obtenir une dose. L'utilisation du dispositif de distribution est donc plus simple et plus sûre. On peut prévoir que le signal est auditif et/ou visuel et/ou vibratoire. Par exemple, le signal peut consister en un compte à rebours affiché sur un écran du dispositif d'assistance et/ou l'énonciation du temps restant et/ou une ou plusieurs vibrations du dispositif d'assistance que l'utilisateur aura appris à associer à un temps restant donné.

Avantageusement, le dispositif d'assistance comprend des moyens de signalement configurés pour émettre au moins un signal donnant une information sur un nombre d'actionnement du dispositif de distribution encore autorisé avant que les moyens de verrouillage ne verrouillent automatiquement le dispositif de distribution.

Ainsi, l'utilisateur est mieux informé de l'état du dispositif de distribution et des possibilités qui lui sont offertes. L'utilisation du dispositif de distribution est ainsi plus simple et plus sûre. On peut prévoir que le signal est auditif et/ou visuel et/ou vibratoire. Par exemple, le signal peut consister en un écran du dispositif d'assistance affichant le nombre d'actionnements du dispositif de distribution encore autorisé et/ou l'énonciation de ce nombre d'actionnements du dispositif de distribution et/ou une ou plusieurs vibrations du dispositif d'assistance correspondant à ce nombre d'actionnements du dispositif de distribution. En outre, dans le cas où le produit est un médicament, le suivi du traitement est facilité puisque l'utilisateur connait le nombre d'actionnements du dispositif de distribution qu'il doit réaliser pour obtenir la quantité de produit correspondant au seuil prédéterminé, et lorsque ce seuil correspond à la dose qu'il doit prendre à chaque prise, il est ainsi informé du nombre d'actionnements du dispositif de distribution nécessaires pour obtenir une dose complète.

L'invention concerne également un procédé d'utilisation d'un dispositif de distribution de produit pharmaceutique ou cosmétique, comprenant les étapes suivantes :
- déverrouillage du dispositif de distribution de manière à permettre la distribution de produit, et
- verrouillage automatique du dispositif de distribution, à l'issue d'une période de temps prédéterminée à partir du déverrouillage du dispositif de distribution.

Le verrouillage automatique à l'issue de la période de temps prédéterminée permet d'éviter que le dispositif de distribution puisse être utilisé jusqu'à la prochaine prise de produit, que ce soit par l'utilisateur auquel le produit est destiné ou non et réduit ainsi le risque de surdosage ou de délivrance accidentelle de produit, notamment lorsque le déverrouillage a eu lieu lui aussi accidentellement.

On peut prévoir que la période de temps prédéterminée est comprise entre 1 et 20 minutes, de préférence entre 5 et 10 minutes.

Avantageusement, le dispositif de distribution est automatiquement verrouillé si une quantité de produit distribuée pendant la période de temps prédéterminée atteint ou dépasse un seuil prédéterminé.

On réduit ainsi le risque de surdosage, notamment dans le cas où le produit est un médicament, en empêchant qu'une dose supérieure à une dose maximale de produit autorisée puisse être délivrée pendant la période de temps prédéterminée. En outre, on réduit le risque de gaspillage de produit dans le cas où le dispositif de distribution serait déverrouillé par accident, par exemple dans un sac ou dans une poche, et que le dispositif de distribution serait actionné à plusieurs reprises accidentellement pendant la période de temps prédéterminée. L'actionnement du dispositif de distribution pourrait ainsi avoir lieu seulement jusqu'à ce que le seuil prédéterminé de produit soit atteint plutôt que jusqu'à l'issue de la période de temps prédéterminée.

### Brève description des figures

Nous allons maintenant présenter un mode de réalisation de l'invention à titre d'exemple non limitatif à l'appui des figures annexés dans lesquelles :

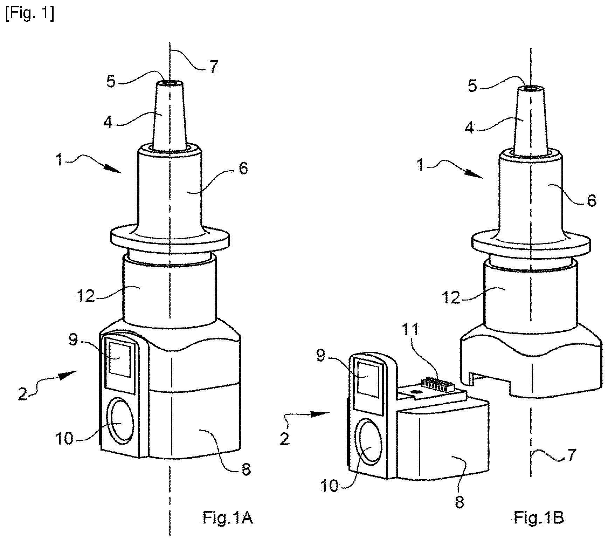

[Fig. 1] les figures 1A et 1B sont des vues en perspective d'un dispositif d'assistance selon l'invention associé à un dispositif de distribution de produit, dans lesquelles une partie jetable et une partie réutilisable de l'ensemble formé par le dispositif d'assistance et le dispositif de distribution sont respectivement représentés assemblés et désassemblés.
[Fig. 2] la figure 2 est une vue en perspective éclatée d'un socle du dispositif d'assistance des figures 1A et 1B.
[Fig. 3]la figure 3 est une vue en perspective éclatée de la partie jetable du dispositif d'assistances des figures 1A et 1B.
[Fig. 4] la figure 4 est une représentation schématique d'un procédé d'enregistrement du dispositif d'assistance des figures 1A et 1B.
[Fig. 5] la figure 5 est une représentation schématique d'un procédé de configuration du dispositif d'assistance des figures 1A et 1B.
[Fig. 6] la figure 6 est une représentation schématique d'un procédé d'enregistrement d'une empreinte digitale pour le déverrouillage du dispositif d'assistance des figures 1A et 1B.
[Fig. 7] la figure 7 est une représentation schématique d'un procédé de mise en route du dispositif d'assistance des figures 1A et 1B.
[Fig. 8] la figure 8 est une représentation schématique d'un mode de réalisation du procédé d'utilisation d'un dispositif de distribution selon l'invention.
[Fig. 9] la figure 9 est une représentation schématique d'un procédé de remplacement d'une partie jetable du dispositif d'assistance des figures 1A et 1B.

### Description détaillée

Dans le mode de réalisation présenté ci-après, on décrit un dispositif d'assistance à l'utilisation d'un dispositif de distribution de produit dans lequel le dispositif de distribution est un spray nasal et le produit est un médicament, notamment un médicament antidouleur à base d'opiacés. Il est possible de prévoir que le dispositif de distribution soit tout autre dispositif de distribution connu de l'homme du métier, par exemple un dispositif de distribution de produit liquide sous forme de goutte. On peut également prévoir que le produit soit tout autre produit compatible pour une utilisation avec un dispositif de distribution de produit, ce produit pouvant être un médicament ou non, par exemple un produit cosmétique.

Le dispositif de distribution 1 auquel est solidarisé le dispositif d'assistance 2 comprend une pompe 4 présentant un embout de distribution 5 et un réservoir 13. Le dispositif de distribution 1 comprend en outre une partie mobile formant un applicateur 6. Cet applicateur 6 est monté mobile à coulissement le long d'un axe longitudinal principal 7 du dispositif de distribution 1 entre une position de repos, dans laquelle l'applicateur 6 est dans sa position la plus distale possible et donc la plus proche de l'embout de distribution 5, et une position d'activation qui est la position minimale que doit occuper l'applicateur 6 pour que la totalité de la dose de produit qu'il est prévu de distribuer lors d'un actionnement du dispositif de distribution 1 soit effectivement distribuée. Il peut s'agir de la position la plus proximale de l'applicateur 6, c'est-à-dire la plus éloignée possible de l'embout de distribution 5. On peut prévoir qu'il s'agit d'une autre position prédéterminée, moins proximale, c'est-à-dire moins éloignée de l'embout de distribution 5. On peut prévoir que tout autre dispositif de distribution soit utilisé en association avec le dispositif d'assistance 2 selon l'invention, à condition que son fonctionnement soit compatible avec celui du dispositif d'assistance 2.

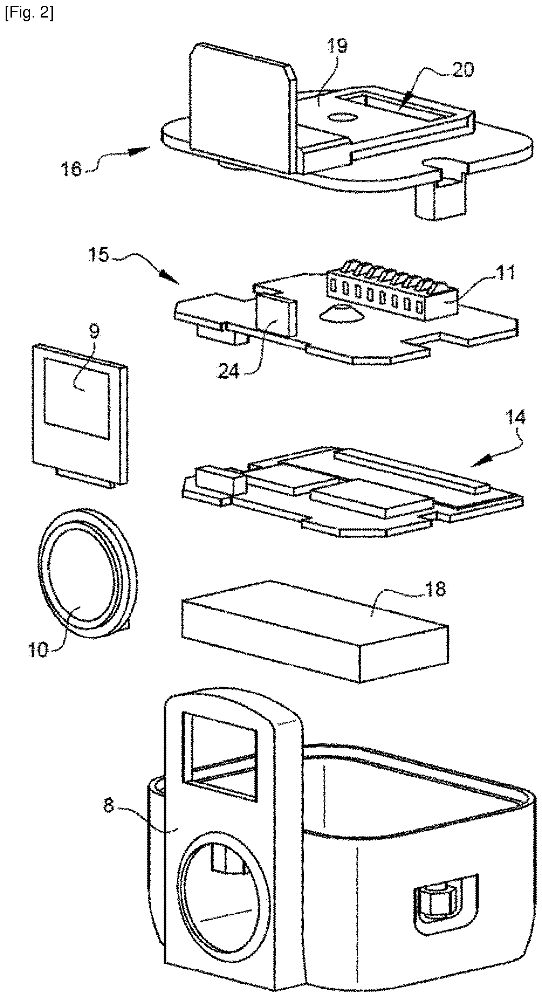

Dans le présent mode de réalisation, le dispositif d'assistance 2 est en plusieurs parties. On peut toutefois prévoir que le dispositif d'assistance 2 est réalisé en une seule partie. Une première partie est formée par un socle 8 réutilisable (voir figure 2). Ce socle 8 comprend, entre autres, des moyens de signalement comprenant un écran 9 apte à afficher un signal visuel, un capteur d'empreinte digitale 10 et des composants électroniques utiles pour le contrôle et le suivi des différentes fonctions du dispositif d'assistance 2. Parmi les composants électroniques, le socle 8 comprend des moyens de communication électronique comprenant dans le cas présent une carte de communication électronique 14 apte à recevoir et transmettre des données depuis et vers un réseau de télécommunication. On peut prévoir que tout autre moyen de communication électronique compatible avec le fonctionnement du dispositif d'assistance 2 tel que décrit ci-après soit utilisé.

Un autre composant électronique compris dans le socle 8 est une carte électronique principale 15 apte à commander les différentes actions des composants électroniques du dispositif d'assistance 2 et à les coordonner entre elles. La carte électronique principale 15 comprend une puce mémoire interne 24 apte à stocker des informations. Une batterie 18 est présente dans le socle 8 et est apte à alimenter les différents composants électroniques du dispositif d'assistance 2. Le socle 8 comprend une paroi supérieure 16. Un relief 19 de connexion à la partie jetable s'étend à partir de cette paroi supérieure 16. Ce relief 19 présente un orifice 20 au travers duquel s'étend une broche électronique 11 portée par la carte électronique principale 15 (voir figure 2).

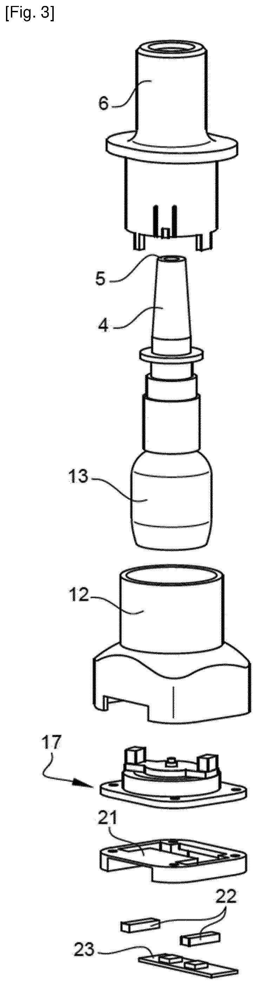

Le dispositif d'assistance 2 comprend en outre des moyens de verrouillage 17 compris dans une base 12 (voir figure 3). La base 12 comprend une paroi inférieure 21 formant un support pour les moyens de verrouillage 17 et présente une face inférieure formant une cavité de forme complémentaire à celle du relief 19 s'étendant à partir du socle 8 et apte à venir en prise avec celle-ci de manière à solidariser la base 12 et le socle 8. À partir de la face inférieure de la paroi inférieure 21 s'étendent également des connecteurs 22 et une puce mémoire jetable 23 (voir figure 3). Lorsque les parties jetable et réutilisable sont assemblées, les connecteurs 22 sont en prise avec la broche électronique 11 qui les mets en communication électronique avec la carte électronique principale 15. En outre, la puce mémoire jetable 23 et elle aussi en prise avec la broche électronique 11 et donc en communication électronique avec la carte électronique principale 15. Le dispositif d'assistance 2 comprend également des moyens de solidarisation (non représentés) au dispositif de distribution 2. Les moyens de solidarisation sont connus en soi et peuvent pas exemple consister en des moyens d'encliquetage, d'emboitement, de vissage, etc. Le dispositif d'assistance 2 comprend des moyens automatisés de vérification qu'au moins une condition prédéterminée est remplie, la condition prédéterminée étant indépendante d'une interaction entre d'une part un utilisateur et d'autre part le dispositif d'assistance 2 ou le dispositif de distribution 1 lors d'une délivrance de produit à l'utilisateur. La carte électronique principale 15 forme au moins une partie des moyens automatisés de vérification. Dans le cas présent, ces moyens automatisés de vérification sont aptes à vérifier au moins une des conditions choisies parmi un amorçage de la pompe 4 du dispositif de distribution 1, une expiration du produit, une concentration du produit, un état d'utilisation du dispositif de distribution 1, une information d'identification du dispositif d'assistance, du produit ou du dispositif de distribution 1 (numéros de lot ou de série non soumis à une demande de retrait du marché). On peut prévoir que ces moyens automatisés sont aptes à vérifier d'autres conditions prédéterminées. La nature des moyens automatisés de vérification dépend de la condition prédéterminé à vérifier. On peut ainsi prévoir que les moyens automatisés de vérification comprennent d'autres éléments que la carte électronique principale 15. On peut prévoir que ces moyens automatisés de vérification sont configurés pour être mis en œuvre automatiquement lors d'une mise en marche du dispositif d'assistance 2. Cette mise en œuvre automatique est commandée par la carte électronique principale 15.

Les moyens de verrouillage 17 peuvent être tout moyen de verrouillage connus de l'homme du métier et compatibles avec le fonctionnement du dispositif de distribution 1. Ces moyens de verrouillage 17 sont aptes à empêcher ou permettre une distribution de produit. Dans le cas présent, les moyens de verrouillage 17 consistent en des moyens mobiles entre une position de verrouillage dans laquelle ils empêchent ou limitent un déplacement de l'applicateur 6 du dispositif de distribution 1 et une position de déverrouillage dans laquelle ils n'empêchent ou ne limitent pas un déplacement de l'applicateur 6. Le déplacement des moyens de verrouillage 17 de la position de verrouillage à la position de déverrouillage et inversement est effectué par un moteur (non représenté) commandé par la carte électronique principale 15. On peut prévoir que tout autre moyen de déplacement des moyens de verrouillage 17 soit utilisé, par exemple des moyens de rappel ou un fil rétractable.

Le dispositif d'assistance 2 comprend également des moyens de mesure d'une quantité de produit distribuée. Ces moyens de mesures sont notamment aptes à mesurer une quantité de produit distribuée pendant la période de temps. Ces moyens peuvent par exemple comprendre des moyens de mesure d'une quantité de produit dans le réservoir 13. En comparant la quantité de produit présente dans le réservoir 13 à deux moments différents, ces moyens de mesure permettent de déduire la quantité de produit distribuée entre ces deux moments. D'autres moyens de mesure de la quantité de produit distribuée connus de l'homme du métier peuvent être utilisés. Par exemple, les moyens de mesure peuvent comprendre des moyens de comptage d'un nombre d'actionnements du dispositif de distribution. Dans le cas où l'actionnement du dispositif de distribution 1 résulte en la distribution d'une quantité prédéterminée de produit, par exemple dans le cas où le dispositif de distribution 1 comprend une pompe 4, le nombre d'actionnements du dispositif de distribution 1 permet de déduire la quantité de produit distribuée.

Le dispositif d'assistance 2 est agencé pour que les moyens de verrouillage 17 restent déverrouillés pendant une période de temps prédéterminée à partir d'un actionnement prédéterminé du dispositif d'assistance 2 puis se verrouillent automatiquement à l'issue de la période de temps et est agencé en outre pour que les moyens de verrouillage 17 se verrouillent automatiquement lorsqu'une quantité de produit distribuée pendant la période de temps prédéterminée atteint ou dépasse un seuil prédéterminé. Cette série d'actions (maintien en position de déverrouillage jusqu'à la fin de la période de temps ou jusqu'à l'atteinte ou le dépassement du seuil de produit distribué puis verrouillage) est commandée par la carte électronique principale 15. Dans le cas présent l'actionnement prédéterminé du dispositif d'assistance 2 correspond à un déverrouillage des moyens de verrouillage 17. Ce déverrouillage peut par exemple être réalisé par authentification d'une empreinte digitale à l'aide du capteur d'empreinte digitale 10 ou par l'entrée d'un code prédéterminé. Dans le cas présent, l'actionnement prédéterminé du dispositif de distribution 1 correspond à un actionnement complet du dispositif de distribution, c'est-à-dire un actionnement entrainant la distribution d'une dose maximale de produit pouvant être délivrée en une seule fois par le dispositif de distribution. On peut prévoir que cet actionnement prédéterminé correspond à un actionnement entrainant la distribution d'une dose incomplète de produit, par exemple entre 60 % et 99 % en volume de la dose maximale de produit pouvant être délivrée en un seul actionnement. Le dispositif d'assistance 2 est configuré pour que les moyens de verrouillage 17 puissent être déverrouillés lorsque la condition prédéterminée est remplie et restent verrouillés lorsque la condition prédéterminée n'est pas remplie. À nouveau, cette série d'action est commandée par la carte électronique principale 15 qui forment ainsi des moyens de contrôle aptes à empêcher ou permettre un déverrouillage des moyens de verrouillage 17.

La période de temps citée précédemment étant une première période de temps, la carte électronique principale 15 formant les moyens de contrôle est configurée pour permettre un déverrouillage des moyens de verrouillage 17 pendant une deuxième période de temps prédéterminée à partir du verrouillage automatique des moyens de verrouillage 17. Le dispositif d'assistance 2 est agencé pour que les moyens de verrouillage 17 se verrouillent automatiquement à l'issue de la deuxième période de temps et pour que les moyens de verrouillage 17 se verrouillent automatiquement lorsqu'une quantité de produit distribuée pendant la deuxième période de temps atteint ou dépasse le seuil prédéterminé.

Le dispositif d'assistance 2 comprend un compteur de temps intégré à la carte électronique principale 15 qui est apte à mesurer le temps écoulé depuis un évènement donné. Notamment, c'est le compteur de temps qui mesure le temps écoulé depuis l'actionnement prédéterminé du dispositif d'assistance 2 et permet à celui-ci de savoir lorsque la première ou deuxième période de temps prédéterminée est écoulée. La première période de temps peut être comprise entre 1 et 60 minutes, de préférence entre 20 et 30 minutes. Dans le cas présent, la période de temps est de 20 minutes. La deuxième période de temps peut être comprise entre 1 et 20 minutes, de préférence entre 5 et 10 minutes. Dans le cas présent, la deuxième période de temps est de 5 minutes.

On peut prévoir que le dispositif d'assistance 2 est configuré pour que les moyens de verrouillage 17 se verrouillent après que le dispositif de distribution 1 a été actionné un nombre de fois prédéterminé pendant la première période de temps. La carte principale 15 recueille les informations sur le nombre d'actionnements effectués pendant le période de temps et commande le verrouillage des moyens de verrouillage 17 lorsque le nombre de fois prédéterminé est atteint durant la période de temps. Ce nombre de fois prédéterminé correspond à la quantité de produit nécessaire pour atteindre ou dépasser le seuil prédéterminé.

La carte électronique principale 15, formant les moyens de contrôle, est configuré pour empêcher un déverrouillage des moyens de verrouillage 17 pendant une troisième période de temps prédéterminée à partir du verrouillage automatique des moyens de verrouillage à l'issue de la deuxième période de temps ou lorsque la quantité de produit distribuée pendant la deuxième période de temps a atteint ou dépassé le seuil prédéterminé. Cette troisième période de temps peut être mesuré par le compteur de temps précédemment cité. Dans le cas présent, cette troisième période de temps est de 4 heures.

L'écran 9 des moyens de signalement est apte à émettre un signal donnant une information sur l'écoulement des première, deuxième et troisième périodes de temps. La carte électronique principale 15 commande le signal qui doit être affiché par l'écran 9. Lorsque les moyens de verrouillage 17 sont déverrouillés par l'utilisateur, la carte électronique principale 15 commande à l'écran 9 d'afficher un minuteur indiquant le temps restant avant que les moyens de verrouillage 17 ne se verrouillent automatiquement si un actionnement prédéterminé du dispositif de distribution 1 n'a pas lieu pendant la première période de temps. Ce temps restant correspond ainsi à l'écoulement de la première période de temps. Lorsque l'utilisateur effectue l'actionnement prédéterminé du dispositif de distribution 1, la carte électronique principale 15 commande un remplacement de cette période de temps par une nouvelle période de temps affichant le temps restant de la seconde période de temps pendant laquelle la carte électronique principale permet un déverrouillage des moyens de verrouillage 17. Il s'agit donc du temps restant avant que les moyens de verrouillage 17 ne puisse plus être déverrouillés jusqu'à l'expiration de la troisième période de temps. La carte électronique principale 15 commande, à la fin de la deuxième période de temps ou, lorsqu'un actionnement du dispositif de distribution 1 a lieu pendant la deuxième période de temps, à la fin de cet actionnement, l'affiche du temps restant de la troisième période de temps sur l'écran. Il s'agit donc du temps restant avant que la carte électronique principale 15 permette un déverrouillage des moyens de verrouillage. On peut prévoir qu'une fois les moyens de verrouillage 17 verrouillés, l'écran 9 affiche un message informant l'utilisateur de cet état. Par exemple, l'écran 9 peut afficher un message tel que « Dispositif verrouillé » ou « Touchez pour déverrouiller ».

Par ailleurs, l'écran 9 des moyens de signalement est apte à émettre au moins un signal donnant une information à l'utilisateur sur un nombre d'actionnements du dispositif de distribution 1 encore autorisé avant que les moyens de verrouillage 17 ne verrouillent automatiquement le dispositif de distribution 1. À nouveau, la carte électronique principale 15 commande le signal qui doit être affiché par l'écran 9. Ce signal peut consister simplement en l'affichage sur l'écran 9 du nombre d'actionnements encore possible avant que la carte électronique principale 15 commande aux moyens de verrouillage 17 de verrouiller le dispositif de distribution 1.

En outre, les moyens de signalement sont aptes à émettre automatiquement au moins un signal lorsque la condition prédéterminée n'est pas remplie. La carte électronique principale 15 commande le signal qui doit être émis par les moyens de signalement. Ce signal peut être visuel et/ou auditif et/ou vibratoire. On peut par exemple prévoir que le signal consiste en un message affiché sur l'écran 9 et indiquant la condition prédéterminée qui n'est pas remplie. Il pourrait également consister en l'énonciation de la condition qui n'est pas remplie par des moyens générateurs de sons.

La carte de communication électronique 14 est apte à effectuer au moins l'une des étapes comprenant au moins recevoir des informations d'un réseau de télécommunication sur une modification de la condition prédéterminée ou d'au moins une des conditions prédéterminées, transmettre des informations au réseau sur la condition prédéterminée ou au moins l'une des conditions prédéterminées.

Le dispositif final, formé par le dispositif de distribution 1 monté sur le dispositif d'assistance 2 comprend une partie jetable (voir figure 3) et une partie réutilisable (voir figure 2). La partie réutilisable est formée par le socle 8 du dispositif d'assistance 2 qui comprend notamment l'écran 9, le capteur d'empreinte digitale 10, la carte électronique principale 15, la carte de communication électronique 14 et la batterie 18. La partie jetable comprend le dispositif de distribution 1 (l'applicateur 6, la pompe 4 et le réservoir 13) ainsi que la base 12, les moyens de verrouillage 17, la puce mémoire jetable 23 et les connecteurs 22 du dispositif d'assistance 2. De cette manière, des économies sont réalisées avec la partie réutilisable qui peut être réutilisée avec d'autres dispositifs de distribution 1.

On décrit maintenant les procédés mis en œuvre au cours de l'utilisation d'un dispositif d'assistance 2 selon l'invention.

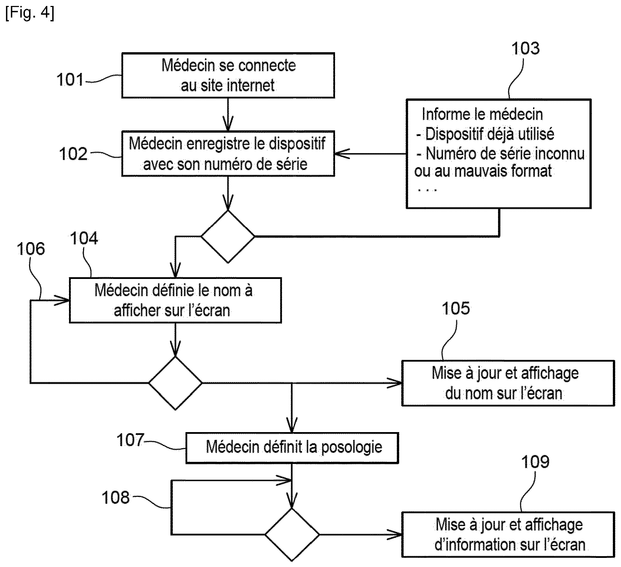

Dans un premier temps, le dispositif d'assistance 2 est enregistré par un médecin, ou toute autre personne compétente pour effectuer cet enregistrement, sur un réseau de télécommunication prévu à cet effet (voir figure 4). Pour cela, le médecin se connecte à ce réseau de télécommunication (étape 101), par exemple un site internet, et y enregistre le dispositif d'assistance 2 en entrant son numéro de série (étape 102). Dans le cas où ce numéro de série est déjà enregistré, n'est pas connu d'une base de données recensant les numéros de séries des différents dispositifs d'assistance 2 qui ont été produits ou n'est pas au bon format, un message est affiché sur une interface utilisateur du réseau pour en informer le médecin qui peut alors prendre les mesures nécessaires (étape 103). Par exemple, il peut se rendre compte qu'il avait renseigné le numéro de série de manière incorrecte et le renseigner à nouveau ou choisir d'utiliser un autre dispositif d'assistance 2. Dans le cas où le numéro de série du dispositif d'assistance 2 correspond à un numéro faisant l'objet d'une demande de retrait du marché, un message est affiché sur l'interface utilisateur pour en informer le médecin. On peut prévoir que cette information est transmise par le réseau de télécommunication au dispositif d'assistance 2 dont la carte électronique principale 15 vérifie que le numéro de série du dispositif d'assistance 2 est bien présent sur cette liste et, en conséquence, la carte électronique principale 15 commande aux moyens de signalement d'émettre un signal indiquant que la condition relative à l'absence du dispositif d'assistance 2 sur une liste de produits faisant l'objet d'une demande de retrait n'est pas remplie. Par exemple, l'écran 9 affiche « Dispositif d'assistance retiré du marché ».

Le médecin choisi ensuite le nom à afficher sur l'écran 9 (étape 104) par exemple lors d'un allumage du dispositif d'assistance 2. Cet affichage est notamment avantageux pour s'assurer avant l'administration du produit que celui-ci est bien destiné à la personne à laquelle il va être administré. Dans le cas où le nom renseigné n'est pas accepté par le réseau de télécommunication, par exemple parce qu'il ne correspond pas à un nom d'un patient présent dans une base de données ou parce qu'il contient des caractères non reconnus par le réseau de télécommunication, il est demandé de rentrer un autre nom (étape 106). Dans le cas où le nom renseigné est accepté, le réseau de télécommunication envoie cette information au dispositif d'assistance 2 et la puce mémoire interne 24 de celui-ci enregistre ce nom et la carte électronique principale 15 commande son affichage sur l'écran 9 (étape 105). Le médecin ou une personne en possession du dispositif d'assistance 2 peut ainsi vérifier que le nom est correctement renseigné.

Une fois que le dispositif d'assistance 2 est enregistré sur le réseau de télécommunication et qu'un patient lui a été attribué, le médecin peut à tout moment définir la posologie du traitement destiné à être pris à l'aide du dispositif d'assistance 2 (étape 107). Il peut notamment définir le médicament utilisé ainsi que son dosage et la fréquence des prises. Le médecin peut modifier cette posologie quand il le souhaite (étape 108). À chaque fois qu'une posologie est définie l'information est transmise au dispositif d'assistance 2 par le réseau de télécommunication via la carte de communication électronique 14 et la carte électronique principale 15 commande un affichage de la nouvelle posologie sur l'écran 9 et l'enregistrement de cette nouvelle posologie sur la puce mémoire interne 24 (étape 109).

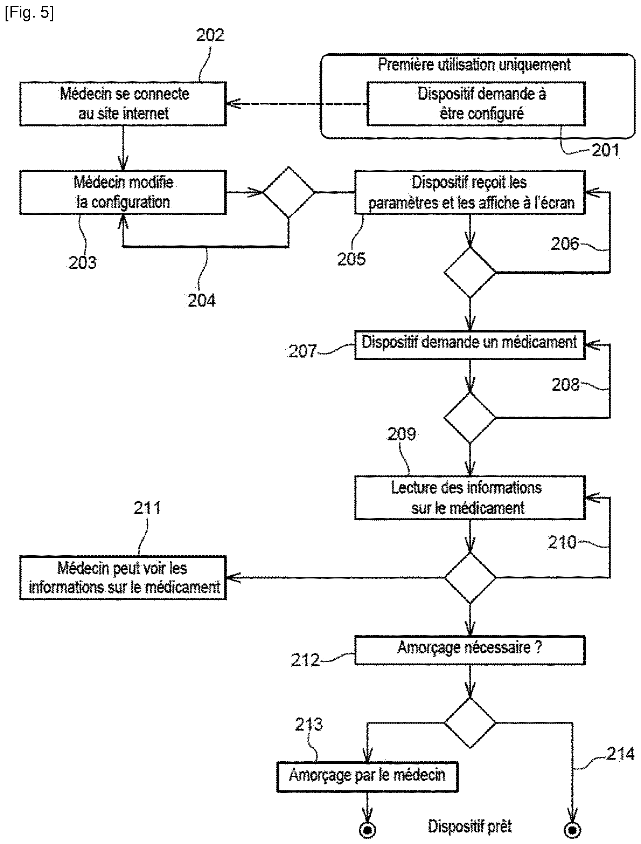

Un procédé de configuration du dispositif d'assistance 2 est décrit ci-après (figure 5).

Lorsque le dispositif d'assistance 2 est allumé pour la première fois, ou en tout cas lorsqu'aucun paramètre de configuration ne lui est associé, la carte électronique principale 15 commande à la carte de communication électronique 14 du dispositif d'assistance 2 d'envoyer un message afin de demander à ce qu'il soit configuré (étape 201). Ce message peut consister en un message affiché sur l'écran 9 du dispositif d'assistance 2 ou sur une interface utilisateur du réseau de télécommunication. Suite à ce message le médecin configure ou modifie les paramètres (étape 203) du dispositif d'assistance 2 en se connectant si besoin au réseau de télécommunication (étape 202). La configuration du dispositif d'assistance 2 peut comprendre l'indication du médicament destiné à être utilisé avec celui-ci, ainsi que sa posologie, que ce soit son dosage ou la fréquence des prises. Le médecin peut également indiquer si le patient peut prendre plus d'une dose à chaque prise, par exemple si le patient peut prendre une double dose. Une fois ces données renseignées sur le réseau de télécommunication et si le dispositif d'assistance 2 est connecté à ce réseau, ces données sont enregistrées sur la puce mémoire interne 24 du dispositif d'assistance 2 et la carte électronique principale 15 commande aux moyens de signalement d'afficher cette nouvelle configuration sur l'écran 9 de manière à ce que la personne en possession du dispositif d'assistance 2, par exemple le médecin ou le patient, puisse prendre connaissance de ces informations (étape 205). Le médecin peut modifier la configuration à tout moment (étape 204). Une fois que le dispositif est configuré le patient peut utiliser le dispositif en le déverrouillant en touchant avec son doigt le capteur d'empreinte digitale 10. Si l'empreinte digitale associée à ce dispositif d'assistance 2 ne correspondant pas à celle du patient, rien ne se passe (étape 206). Si l'empreinte digitale correspond, la carte électronique principale 15 commande à l'écran 9 d'afficher un message demandant l'ajout du dispositif de distribution 1 comprenant le médicament (étape 207). Ce message reste affiché tant que le dispositif de distribution 1 contenant le médicament n'est pas monté sur le dispositif d'assistance 2 (étape 208). Ce montage peut se faire selon des méthodes connues de l'homme du métier. Le dispositif d'assistance 2 comprend des moyens de détection aptes à détecter la présence ou l'absence d'un dispositif de distribution 1 monté sur le dispositif d'assistance 2. Ces moyens de détection peuvent consister en tous moyens connus en eux-mêmes. Ces moyens de détection sont aptes à transmettre une information à la carte électronique principale 15, cette information indiquant si un dispositif de distribution 1 est monté ou non sur le dispositif d'assistance 2. Une fois que le dispositif d'assistance 2 a détecté qu'un dispositif de distribution 1 contenant un médicament a été monté, il lit les informations de ce dispositif de distribution 1 (étape 209), ces informations pouvant par exemple être lues au moyen de la technologie RFID (pour « radio frequency identification », aussi appelé radio-identification). Par exemple, on peut prévoir que le dispositif de distribution 1 comprend une radio-étiquette (ou « tag RFID » en anglais) contenant des informations sur le médicament que le dispositif de distribution 1 comprend (principe actif ou nom commerciale du médicament, concentration du médicament, quantité de produit contenue dans le réservoir 13 etc.). Cette radio-étiquette est lue par un lecteur compris dans le dispositif d'assistance 2. La carte électronique principale 15 compare les informations lues sur la radio-étiquette avec les paramètres renseignés par le médecin et qui ont été enregistrés sur la puce mémoire interne 24. Si les informations lues sur la radio-étiquette et les paramètres enregistrés sur la puce mémoire interne 24 sont différentes, c'est-à-dire que les conditions prédéterminées relatives à ces paramètres ne sont pas remplies, le dispositif d'assistance 2 revient à l'étape précédente (étape 210) et la carte électronique principale 15 commande aux moyens de signalement l'affichage d'un message sur l'écran 9 afin d'informer l'utilisateur que le médicament associé au dispositif d'assistance 2 ne présente pas au moins une des caractéristiques requises. On peut prévoir qu'un message d'alerte soit envoyé au médecin via le réseau de télécommunication. Si les informations lues sur la radio-étiquette et les paramètres enregistrés correspondent, un message est envoyé sur le réseau de télécommunication et peut être consulté par le médecin pour que celui-ci voit les informations du nouveau médicament installé sur le dispositif d'assistance 2 (étape 211). La radio-étiquette comprend aussi une information relative à une autre condition prédéterminée qui concerne la nécessité ou non de réaliser un amorçage du dispositif de distribution 1 comprenant le médicament, notamment lorsque ce dispositif de distribution 1 comprend une pompe 4. La condition prédéterminée à remplir consiste donc en ce que la pompe 4 du dispositif de distribution 1 a été amorcée. Si un amorçage est nécessaire, c'est-à-dire que la condition prédéterminée correspondante n'est pas remplie, la carte électronique principale 15 commande à l'écran 9 d'afficher un message afin que l'utilisateur en soit informé. On peut prévoir que le message précise également la personne que l'utilisateur doit contacter pour réaliser l'amorçage, par exemple le médecin (étape 213). Si aucun amorçage n'est nécessaire, soit aucun message n'est affiché sur l'écran 9, soit un message informant que le dispositif de distribution 1 est prêt à être utilisé est affiché sur l'écran 9 (étape 214).

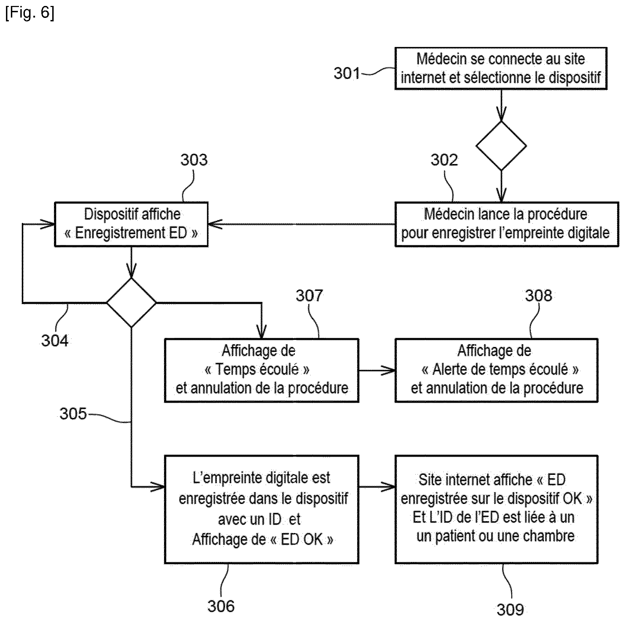

Dans le cas où le déverrouillage du dispositif est réalisé par authentification d'une empreinte digitale à l'aide du capteur d'empreinte digitale 10, cette empreinte doit au préalable être enregistrée (voir figure 6).

Pour cela, le médecin se connecte au même réseau de télécommunication que précédemment et sélectionne le dispositif d'assistance 2 auquel il souhaite associer une empreinte digitale (étape 301). Il peut rechercher le dispositif d'assistance 2 dans la base de données soit par son numéro de série, soit par le nom du patient qui a déjà été enregistré et associé au dispositif d'assistance 2 selon le procédé décrit précédemment.

Si le dispositif d'assistance 2 n'est pas connecté au réseau de télécommunication, un message informe le médecin qu'il ne peut pas se connecter au dispositif d'assistance 2. Le médecin peut alors soit allumer le dispositif d'assistance 2 de manière à ce qu'il se connecte au réseau de télécommunication, soit attendre qu'une autre personne, par exemple le patient, le fasse. Une fois que le dispositif d'assistance 2 est connecté au réseau de télécommunication, le médecin lance le processus d'enregistrement de l'empreinte digitale (étape 302). La carte électronique principale 15 commande à l'écran 9 d'afficher alors « Enregistrement ED » (étape 303) et le patient pose un de ses doigts, par exemple un index, sur le capteur d'empreinte digitale 10.

Si le dispositif d'assistance 2 n'arrive pas à enregistrer correctement l'empreinte digitale, par exemple si le patient a bougé son doigt au cours de l'enregistrement, l'écran 9 continue d'afficher le même message (étape 304). Si aucune empreinte n'a pu être correctement enregistrée pendant une période de temps donnée, par exemple une minute, la carte électronique principale 15 commande aux moyens de signalement d'afficher sur l'écran 9 un message pour en informer le patient, par exemple « Temps écoulé » et la procédure d'enregistrement de l'empreinte digitale est annulée (étape 307). Un message d'alerte est également envoyé sur le réseau de télécommunication afin notamment que le médecin en soit averti (étape 308). Ce message peut être « Alerte de temps écoulé ».

Si une empreinte digitale est correctement enregistrée dans le temps donné, un numéro identifiant est associé à cette empreinte (ID empreinte) et la carte électronique principale 15 l'associe à un identifiant du patient auquel elle correspond, par exemple un numéro ID patient ou son nom, et commande à la carte de communication électronique 14 d'envoyer un message au réseau de télécommunication afin que ces informations (ID empreinte et ID patient) soient enregistrées (étapes 306 et 309). En outre, l'écran 9 affiche alors le message « ED OK ». Sur une interface utilisateur du réseau de télécommunication le message « ED enregistrée sur le dispositif OK » est affiché afin d'informer le médecin que l'enregistrement de l'empreinte digitale du patient et son association avec le dispositif d'assistance 2 a bien réussi. L'information est alors enregistrée dans la base de données liant le dispositif d'assistance 2 au patient. Les données relatives à l'empreinte digitale du patient étant enregistrées dans la puce mémoire interne 24 du dispositif d'assistance 2, le patient peut déverrouiller les moyens de verrouillage 17 même lorsque le dispositif d'assistance 2 n'est pas connecté au réseau de télécommunication.

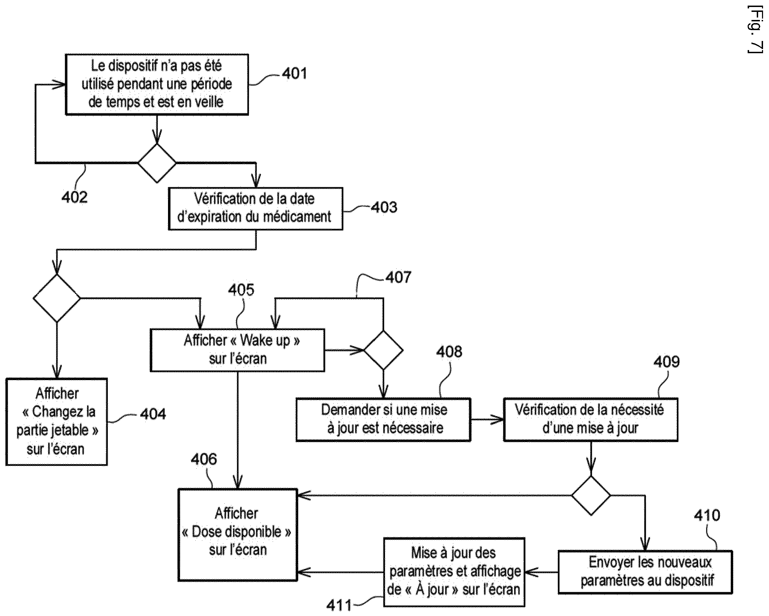

On décrit ci-après un procédé de sortie d'un mode veille du dispositif d'assistance 2 (voir figure 7).

Dans le cas où le dispositif d'assistance 2 n'a pas été utilisé pendant une période de temps prolongée, par exemple pendant plusieurs heures, celui-ci entre en mode veille (étape 401). Il est nécessaire de sortir de ce mode veille pour pouvoir utiliser le dispositif de distribution 1 associé au dispositif d'assistance 2. Pour cela, l'utilisateur appuie son doigt dont l'empreinte digitale a été enregistré sur le capteur d'empreinte digitale 10. Des moyens de contrôle connectés à la carte électronique principale 15 vérifient que l'empreinte digitale captée par le capteur d'empreinte digitale 10 correspond bien au numéro identifiant associé à l'empreinte digitale enregistrée sur la puce mémoire interne 24 et donc correspond à une personne autorisée à utiliser le dispositif de distribution 1 associé au dispositif d'assistance 2. Si l'empreinte digitale ne correspond pas à une empreinte digitale enregistrée, le dispositif d'assistance 2 reste en mode veille et les moyens de verrouillage 17 du dispositif d'assistance 2 restent verrouillés (étape 402). Si l'empreinte digitale correspond à au moins une empreinte digitale enregistrée, la carte électronique principale 15 vérifie si une date prédéterminée, par exemple une date d'expiration du produit contenu dans le dispositif de distribution 1, est dépassée (étape 403). On peut prévoir que la date d'expiration est indiquée sur la radio-étiquette qui est lue par le dispositif d'assistance 2. Si la date d'expiration est dépassée, la carte électronique principale 15 commande à l'écran 9 d'afficher un message invitant à changer la partie jetable, le message indiquant par exemple « Changez la partie jetable » (étape 404). Si la date d'expiration n'est pas dépassée, la carte électronique principale 15 commande à l'écran 9 d'afficher un message indiquant que le dispositif d'assistance 2 est en cours de sortie du mode veille, par exemple l'écran 9 affiche « Wake up » (étape 405). Suite à cela, soit le dispositif d'assistance 2 affiche un message sur l'écran 9 afin d'indiquer que le dispositif de distribution 1 est prêt à être utilisé, par exemple un message indiquant « Dose disponible » ou « Prêt » et la carte électronique principale 15 commande un déverrouillage des moyens de verrouillage 17 (étape 406), soit le dispositif d'assistance 2 tente d'accéder au réseau de télécommunication afin de vérifier si des mises à jour des paramètres du dispositif d'assistance 2 sont disponibles (étape 408). Dans ce dernier cas, le dispositif d'assistance 2 tente d'accéder au réseau de télécommunication et affiche « Wake up » sur l'écran 9 tant qu'il n'y parvient pas (étape 407). Une fois qu'il y parvient, des moyens de contrôle vérifient si une mise à jour du dispositif d'assistance 2 est nécessaire (étape 409). Si aucune mise à jour n'est nécessaire, le dispositif de distribution 1 est prêt à être utilisé et la carte électronique principale 15 commande à l'écran 9 d'afficher un message en ce sens, par exemple « Dose disponible » ou « Prêt » (étape 406), et la carte électronique principale 15 commande un déverrouillage des moyens de verrouillage 17. Si une mise à jour est nécessaire, par exemple une mise à jour d'un paramètre de configuration du dispositif d'assistance 2 tel qu'une nouvelle posologie, les moyens de télécommunications envoient les données comprenant ces nouveaux paramètres au dispositif d'assistance 2 et la carte électronique principale 15 commande un enregistrement de ces données sur la puce mémoire interne 24 (étape 410). L'écran 9 affiche alors un message indiquant que le dispositif d'assistance 2 a été mis à jour, par exemple l'écran 9 affiche « À jour » (étape 411). Ensuite, la carte électronique principale 15 commande à l'écran 9 d'afficher un message indiquant que le dispositif de distribution 1 est prêt à être utilisé, par exemple « Dose disponible » ou « Prêt », et commande un déverrouillage des moyens de verrouillage 17 (étape 406).

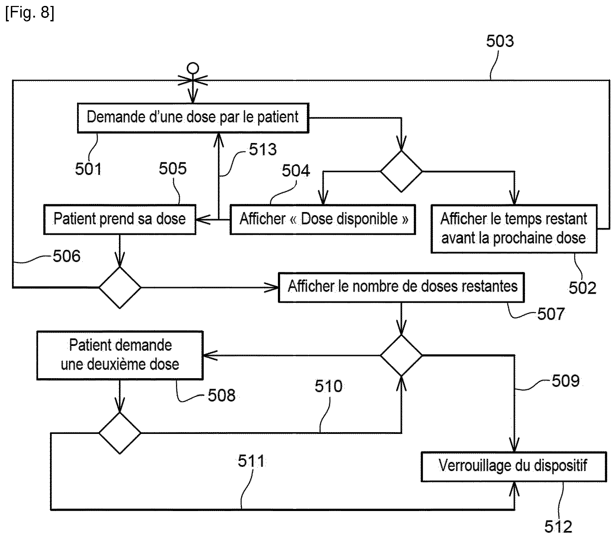

On décrit maintenant un procédé d'utilisation du dispositif de distribution 1 selon l'invention (voir figure 8).

Dans le présent exemple, le produit est un médicament antidouleurs, le patient utilise donc le dispositif d'assistance 2 et le dispositif de distribution 1 lorsqu'il souhaite soulager une douleur. Pour déverrouiller le dispositif, le patient appuie son empreinte digitale sur le capteur d'empreinte 10 après que celle-ci ait été préalablement enregistrée selon le procédé décrit précédemment (étape 501). Dans un premier temps, le dispositif d'assistance 2 vérifie que le patient a le droit de prendre une dose en fonction des caractéristiques renseignées par le médecin lors de la configuration du dispositif. Notamment, la carte électronique principale 15 vérifient que la troisième période de temps a expiré. Si le patient, au regard de la configuration effectuée par le médecin, n'est pas autorisé à prendre une dose à cet instant, par exemple parce que la troisième période de temps n'a pas expiré, la carte électronique principale 15 empêchent un déverrouillage des moyens de verrouillage 17 et commandent à l'écran 9 d'afficher le temps restant avant que le patient puisse prendre une dose, c'est-à-dire le temps restant de la troisième période de temps (étape 502 et 503). Par ailleurs, si l'empreinte digitale du patient ne correspond pas à celle qui a été enregistrée, alors la carte électronique principale 15 commande également aux moyens de verrouillage 17 de rester verrouillés. On peut prévoir que la carte électronique principale 15 commande à l'écran 9 d'afficher un message informant le patient que l'empreinte digitale ne correspond pas à l'empreinte digitale enregistrée afin qu'il soit informé de la raison du non-déverrouillage des moyens de verrouillage 17. Si l'empreinte digitale du patient correspond et que la posologie renseignée par le médecin permet la prise de médicament, par exemple si la troisième période de temps a expiré, la carte électronique principale 15 commande un déverrouillage des moyens de verrouillage 17 et commande à l'écran 9 d'afficher un message indiquant que la prise de dose est possible, par exemple « Dose disponible » (étape 504).

Une fois que les moyens de verrouillage 17 sont déverrouillés, l'actionnement prédéterminé du dispositif de distribution 1 est possible. Le compteur de temps décompte la première période de temps à partir de ce déverrouillage. Si la période de temps mesurée par le compteur de temps atteint ou dépasse la première période de temps prédéterminée sans que l'actionnement prédéterminé n'ait eu lieu, la carte électronique principale 15 commande un verrouillage des moyens de verrouillage 17. Dans le cas présent, la première période de temps prédéterminée est de 5 minutes.

Ainsi, le patient doit prendre une première dose de médicament pendant la première période de temps prédéterminée pour éviter que les moyens de verrouillage 17 ne se reverrouillent (étape 513). Pour cela, après avoir positionné convenablement le dispositif de distribution 1 (ici, s'agissant d'un spray nasal, le patient introduit l'embout de distribution 5 dans une de ses narines) le patient actionne le dispositif de distribution 1 conformément à l'actionnement prédéterminé (étape 505). Comme indiqué précédemment, dans le cas présent, cet actionnement prédéterminé consiste en un actionnement entrainant la distribution d'une dose maximale de produit pouvant être délivrée en une seule fois par le dispositif de distribution 1. Après la distribution de cette première dose de médicament, la carte électronique principale 15 commande aux moyens de verrouillage 17 de se verrouiller et commande à l'écran 9 d'afficher le nombre de doses pouvant encore être prises ainsi que le temps restant de la deuxième période de temps pendant laquelle l'utilisateur a la possibilité de déverrouiller à nouveau les moyens de verrouillage 17 afin d'obtenir une deuxième dose de produit (étape 507). Ainsi, durant cette période, le patient peut évaluer si une dose supplémentaire est nécessaire afin de soulager la douleur qu'il ressent. À l'issue de la deuxième période de temps, la carte électronique principale 15 empêche un déverrouillage des moyens de verrouillage 17 jusqu'à l'expiration de la troisième période de temps (étape 509). Si le patient souhaite obtenir une deuxième dose, il déverrouille les moyens de verrouillage 17 de la même façon que décrite précédemment (étape 508) puis actionne le dispositif de distribution 1 afin d'obtenir la dose. Une fois la dose distribuée, la carte électronique principale 15 commande un verrouillage des moyens de verrouillage 17 (étape 512). On peut prévoir que le patient puisse demander plus d'une dose supplémentaire pendant la deuxième période de temps (étape 510). Dans ce cas, une fois la deuxième dose de produit distribuée et les moyens de verrouillage 17 verrouillés, le patient peut déverrouiller à nouveau les moyens de verrouillage 17 de la même façon que décrite précédemment, à condition que la deuxième période de temps n'ait pas encore expirée.

Dans le cas présent, le dispositif d'assistance 2 est configuré pour que les moyens de verrouillage 17 se verrouillent après que le dispositif de distribution 1 a été actionné une fois pendant la première période de temps, ce qui signifie que la quantité de produit distribuée lors de l'actionnement du dispositif de distribution 1 atteint ou dépasse le seuil prédéterminé, c'est-à-dire qu'elle correspond à la dose devant être prise par le patient lors de chaque prise de médicament. On peut prévoir que cette dose soit atteinte suite à plusieurs actionnements du dispositif de distribution 1.

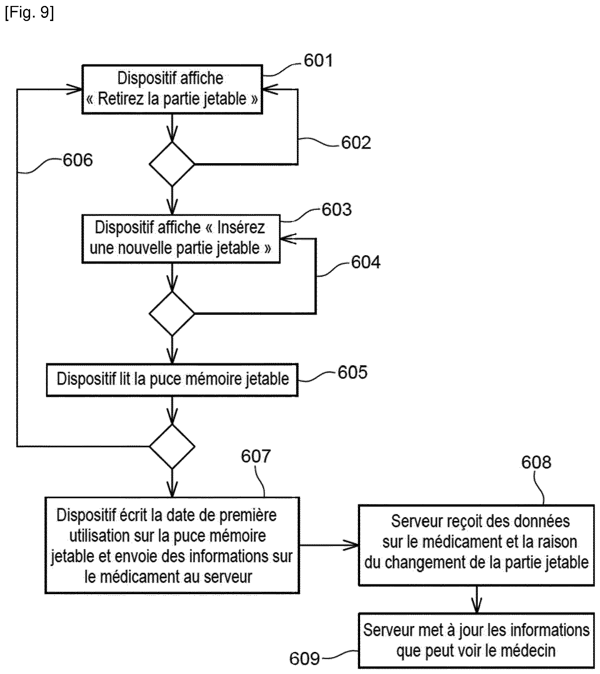

On décrit ci-après un procédé de remplacement de la partie jetable de l'ensemble formé par le dispositif d'assistance 2 et le dispositif de distribution 1 (figure 9).

Comme indiqué précédemment, la partie jetable comprend le dispositif de distribution 1 ainsi que la base 12 et les moyens de verrouillage 17 du dispositif d'assistance 2. Il existe plusieurs situations dans lesquelles il est souhaitable de remplacer la partie jetable, notamment lorsque le réservoir 13 du dispositif de distribution 1 est vide, lorsqu'un autre produit (par exemple un autre médicament) doit être utilisé, ou lorsque le produit a dépassé une date prédéterminée, par exemple une date d'expiration.

Le dispositif d'assistance 2 est configuré pour détecter les cas dans lesquels un remplacement de la partie jetable est nécessaire. Cette détection peut être réalisée par la carte électronique principale 15 ou un autre élément des moyens automatisés de vérification d'une condition prédéterminée. Par exemple et comme indiqué précédemment, le dispositif d'assistance 2 est apte à lire une information présente sur la radio-étiquette du dispositif de distribution 1 relative à la date d'expiration du produit contenu dans le réservoir 13. En outre, le dispositif d'assistance 2 peut comprendre des capteurs aptes à mesurer un niveau de remplissage du réservoir 13 du dispositif de distribution 1. Ainsi, lorsque le dispositif détecte une situation dans laquelle un remplacement de la partie jetable est nécessaire, la carte électronique principale 15 commande à l'écran 9 des moyens de signalement d'afficher un message informant que ce remplacement doit avoir lieu, par exemple l'écran 9 affiche « Retirez la partie jetable » (étape 601). Le dispositif d'assistance 2 est configuré pour afficher ce message tant que la partie jetable n'a pas été séparée de la partie réutilisable (étape 602). Une fois que la partie jetable a été retirée, la carte électronique principale 15 commande à l'écran 9 d'afficher un message demandant d'insérer une nouvelle partie jetable, par exemple l'écran 9 affiche « Insérez une nouvelle partie jetable » (étape 603). Ce message est affiché tant que le détecteur de présence d'une partie jetable ne détecte pas la présence d'une partie jetable associée à la partie réutilisable (étape 604). Une fois qu'une nouvelle partie jetable a été insérée, le dispositif d'assistance 2 lit la puce mémoire jetable 23 et les moyens automatisés de vérification vérifient qu'au moins deux conditions sont remplies (étape 605). Ces actions sont pilotées par la carte électronique principale 15 qui forme au moins une partie des moyens automatisés de vérification. La première condition consiste en ce que la partie jetable ajoutée est neuve, c'est-à-dire qu'elle n'a pas encore été utilisée en association avec une partie réutilisable. On peut prévoir que les moyens automatisés de vérification vérifient uniquement si le dispositif de distribution 1 de la partie jetable est neuf. Cette information peut être vérifiée en lisant la puce mémoire 23 puisque celle-ci enregistre les données liées à une première utilisation de la partie jetable. La deuxième condition consiste en ce que la concentration du médicament contenu dans le réservoir 13 correspond aux paramètres indiqués par le médecin lors de la configuration du dispositif d'assistance 2. Dans le cas où au moins une de ces conditions n'est pas remplie, la carte électronique principale 15 commande l'affichage sur l'écran 9 d'un message demandant de retirer la partie jetable, par exemple « Retirez la partie jetable » (étape 606). Dans le cas où au moins les deux conditions sont remplies, la carte électronique principale 15 commande l'écriture de la date de première utilisation sur la puce mémoire jetable 23 présente dans la partie jetable et la carte de communication électronique 14 envoie les informations sur le médicament présent dans le dispositif de distribution 1 au réseau de télécommunication (étape 607). Le réseau de télécommunication reçoit et enregistre les informations sur le nouveau médicament ainsi que sur les raisons du remplacement de la partie jetable (étape 608). Ces informations sont mises à jour et consultables par le médecin sur le réseau de télécommunication (étape 609). Le médecin est ainsi mieux informé du suivi du traitement par le patient.

L'invention n'est pas limitée au mode de réalisation présenté et d'autres modes de réalisation apparaîtront clairement à l'homme du métier.

## Revendications

1. Dispositif d'assistance (2) à l'utilisation d'un dispositif de distribution (1) d'un produit, le dispositif d'assistance (2) comprenant :
- des moyens de solidarisation au dispositif de distribution (1),
- des moyens de verrouillage (17) du dispositif de distribution (1) aptes à empêcher ou permettre une distribution de produit, et
- des moyens de mesure d'une quantité de produit distribuée par le dispositif de distribution (1),
le dispositif d'assistance (2) étant agencé pour que les moyens de verrouillage (17) restent déverrouillés pendant une première période de temps prédéterminée à partir d'un actionnement prédéterminé du dispositif d'assistance (2) puis se verrouillent automatiquement à l'issue de la première période de temps, et étant agencé en outre pour que les moyens de verrouillage (17) se verrouillent automatiquement lorsqu'une quantité de produit distribuée pendant la première période de temps prédéterminée atteint ou dépasse un seuil prédéterminé,
**caractérisé en ce que** le dispositif d'assistance (2) comprend en outre des moyens de contrôle (15) aptes à empêcher ou permettre un déverrouillage des moyens de verrouillage (17), les moyens de contrôle (15) étant configurés pour permettre un déverrouillage des moyens de verrouillage (17) pendant une deuxième période de temps prédéterminée à partir du verrouillage automatique des moyens de verrouillage (17),
le dispositif d'assistance (2) étant agencé pour que les moyens de verrouillage (17) se verrouillent automatiquement à l'issue de la deuxième période de temps et pour que les moyens de verrouillage (17) se verrouillent automatiquement lorsqu'une quantité de produit distribuée pendant la deuxième période de temps atteint ou dépasse le seuil prédéterminé .

2. Dispositif d'assistance (2) selon la revendication précédente, dans lequel la première période de temps est comprise entre 1 et 30 minutes, de préférence entre 5 et 10 minutes.

3. Dispositif d'assistance (2) selon l'une quelconque des revendications précédentes, dans lequel la deuxième période de temps est comprise entre 1 et 60 minutes, de préférence entre 10 et 30 minutes.

4. Dispositif d'assistance (2) selon l'une quelconque des revendications précédentes, dans lequel les moyens de contrôle (15) sont configurés pour empêcher un déverrouillage des moyens de verrouillage (17) pendant une troisième période de temps prédéterminée à partir du verrouillage automatique des moyens de verrouillage (17) à l'issue de la deuxième période de temps ou lorsque la quantité de produit distribuée pendant la deuxième période de temps a atteint ou dépassé le seuil prédéterminé.

5. Dispositif d'assistance (2) selon l'une quelconque des revendications précédentes, les moyens de mesure de la quantité de produit distribuée comprennent un compteur d'un nombre d'actionnements prédéterminé du dispositif de distribution (1).

6. Dispositif d'assistance (2) selon l'une quelconque des revendications précédentes, comprenant des moyens de signalement (9) configurés pour émettre au moins un signal donnant une information sur l'écoulement d'au moins une des première, deuxième et troisième périodes de temps.

7. Dispositif d'assistance (2) selon l'une quelconque des revendications précédentes, comprenant des moyens de signalement (9) configurés pour émettre au moins un signal donnant une information sur un nombre d'actionnement du dispositif de distribution (1) encore autorisé avant que les moyens de verrouillage (17) ne verrouillent automatiquement le dispositif de distribution (1).

## Patentansprüche

1. Vorrichtung zur Unterstützung (2) der Verwendung einer Vorrichtung zur Abgabe (2) eines Produkts, wobei die Unterstützungsvorrichtung (2) aufweist:
- Mittel zur festen Verbindung mit der Abgabevorrichtung (1),
- Verriegelungsmittel (17) der Abgabevorrichtung (1), die geeignet sind, eine Abgabe des Produkts zu verhindern oder zu ermöglichen, und
- Mittel zum Messen einer von der Abgabevorrichtung (1) abgegebenen Produktmenge,
wobei die Unterstützungsvorrichtung (2) so konfiguriert ist, dass die Verriegelungsmittel (17) während einer ersten vorbestimmten Zeitdauer ab einer vorbestimmten Betätigung der Unterstützungsvorrichtung (2) entriegelt bleiben und sich dann nach Ablauf der ersten Zeitdauer automatisch verriegeln, und ferner so konfiguriert ist, dass die Verriegelungsmittel (17) sich automatisch verriegeln, wenn eine während der ersten vorbestimmten Zeitdauer abgegebene Produktmenge einen vorbestimmten Schwellenwert erreicht oder überschreitet,
**dadurch gekennzeichnet, dass** die Unterstützungsvorrichtung (2) ferner Steuermittel (15) aufweist, die geeignet sind, eine Entriegelung der Verriegelungsmittel (17) zu verhindern oder zuzulassen, wobei die Steuermittel (15) so konfiguriert sind, dass sie eine Entriegelung der Verriegelungsmittel (17) während einer zweiten vorbestimmten Zeitperiode ab der automatischen Verriegelung der Verriegelungsmittel (17) zulassen,
wobei die Unterstützungsvorrichtung (2) so konfiguriert ist, dass die Verriegelungsmittel (17) nach Ablauf der zweiten Zeitperiode automatisch verriegelt werden und dass die Verriegelungsmittel (17) automatisch verriegelt werden, wenn eine während der zweiten Zeitperiode ausgegebene Produktmenge den vorbestimmten Schwellenwert erreicht oder überschreitet.

2. Unterstützungsvorrichtung (2) nach dem vorhergehenden Anspruch, wobei der erste Zeitabschnitt zwischen 1 und 30 Minuten, vorzugsweise zwischen 5 und 10 Minuten, beträgt.

3. Unterstützungsvorrichtung (2) nach einem der vorhergehenden Ansprüche, wobei die zweite Zeitperiode zwischen 1 und 60 Minuten, vorzugsweise zwischen 10 und 30 Minuten, beträgt.

4. Unterstützungsvorrichtung (2) nach einem der vorhergehenden Ansprüche, wobei die Steuermittel (15) so konfiguriert sind, dass sie eine Entriegelung der Verriegelungsmittel (17) während einer dritten vorbestimmten Zeitdauer ab der automatischen Verriegelung der Verriegelungsmittel (17) am Ende der zweiten Zeitdauer verhindern oder wenn die während der zweiten Zeitdauer ausgegebene Produktmenge den vorbestimmten Schwellenwert erreicht oder überschritten hat.

5. Unterstützungsvorrichtung (2) nach einem der vorhergehenden Ansprüche, wobei die Mittel zur Messung der Menge des abgegebenen Produkts einen Zähler für eine vorbestimmte Anzahl von Betätigungen der Abgabevorrichtung (1) aufweisen.

6. Unterstützungsvorrichtung (2) nach einem der vorhergehenden Ansprüche, mit Signalmitteln (9), die so konfiguriert sind, dass sie mindestens ein Signal aussenden, das eine Information über den Ablauf von mindestens einer der ersten, zweiten und dritten Zeitperioden liefert.

7. Unterstützungsvorrichtung (2) nach einem der vorhergehenden Ansprüche, mit Signalmitteln (9), die so konfiguriert sind, dass sie mindestens ein Signal ausgeben, das eine Information über eine Anzahl von Betätigungen der Abgabevorrichtung (1) liefert, die noch zulässig sind, bevor die Verriegelungsmittel (17) die Abgabevorrichtung (1) automatisch verriegeln.

## Claims

1. Assistance device (2) for use of a product dispensing device (1), the assistance device (2) comprising:
- engaging means to the dispensing device (1),
- locking means (17) the dispensing device (1) which are capable of preventing or permitting dispensing of the product, and
- measurement means for measuring a quantity of product dispensed by the dispensing device (1),
the assistance device (2) being arranged such that the locking means (17) remain unlocked for a first predetermined period of time starting from a predetermined activation of the dispensing device (2) and then lock automatically at the end of the first period of time, and being further arranged such that the locking means (17) lock automatically when a quantity of product dispensed during the first predetermined period of time reaches or exceeds a predetermined threshold, **characterized in that** the assistance device (2) further comprises control means (15) capable of preventing or permitting unlocking of the locking means (17), the control means (15) being configured to permit unlocking of the locking means (17) during a second predetermined period of time starting from the automatic locking of the locking means (17),
the assistance device (2) being arranged such that the locking means (17) lock automatically at the end of the second period of time and such that the locking means (17) lock automatically when a quantity of product dispensed during the second period of time reaches or exceeds the predetermined threshold.

2. Assistance device (2) according to the preceding claim, wherein the first period of time is between 1 and 30 minutes, preferably between 5 and 10 minutes.

3. Assistance device (2) according to any one of the preceding claims, wherein the second period of time is between 1 and 60 minutes, preferably between 10 and 30 minutes.

4. Assistance device (2) according to any of the preceding claims, wherein the control means (15) are configured to prevent the locking means (17) from unlocking during a third predetermined period of time starting from the automatic locking of the locking means (17) at the end the second period of time or when the quantity of product dispensed during the second period of time has reached or exceeded the predetermined threshold.

5. Assistance device (2) according to any one of the preceding claims, wherein the measurement means for measuring the quantity of product dispensed comprise a counter for counting a number of predetermined activations of the dispensing device (1).

6. Assistance device (2) according to any one of the preceding claims, comprising signalling means (9) configured to emit at least one signal providing information concerning the countdown of at least one of the first, second and third periods of time.

7. Assistance device (2) according to any one of the preceding claims, comprising signalling means (9) configured to emit at least one signal providing information concerning a number of activations of the dispensing device (1) that are still authorized before the locking means (17) automatically lock the dispensing device (1).
